**Europäisches Patentamt**

**European Patent Office**

**Office européen des brevets**

(11) Veröffentlichungsnummer: **0 292 587**
**B1**

(12) **EUROPÄISCHE PATENTSCHRIFT**

(45) Veröffentlichungstag der Patentschrift:
07.11.90

(51) Int. Cl.⁵: **A61M 29/00, A61F 2/06**

(21) Anmeldenummer: 87107638.6

(22) Anmeldetag: 26.05.87

(54) Katheter zum Herstellen oder Erweitern von Verbindungen zu oder zwischen Körperhohlräumen.

(43) Veröffentlichungstag der Anmeldung:
30.11.88 Patentblatt 88/48

(45) Bekanntmachung des Hinweises auf die Patenterteilung:
07.11.90 Patentblatt 90/45

(84) Benannte Vertragsstaaten:
AT BE CH DE ES FR GB IT LI NL SE

(56) Entgegenhaltungen:
EP-A- 0 183 372
EP-A- 0 221 570
DE-U- 1 706 147
FR-A- 2 391 709
FR-A- 2 525 896

(73) Patentinhaber: Strecker, Ernst Peter, Dr.-med.Prof.,
Vierordtstrasse 7a, D-7500 Karlsruhe 41(DE)

(72) Erfinder: Strecker, Ernst Peter, Dr.-med.Prof.,
Vierordtstrasse 7a, D-7500 Karlsruhe 41(DE)

(74) Vertreter: Geitz, Heinrich, Dr.-Ing.,
Postfach 2708 Kaiserstrasse 156,
D-7500 Karlsruhe 1(DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung bezieht sich auf eine Vorrichtung zum Herstellen oder Erweitern von Verbindungen zu oder zwischen Körperhohlräumen, mit einem Katheter, der einen infolge Druckmedienbeaufschlagung über ein Lumen radial aufweitbaren Ballonabschnitt besitzt, und mit einer auf dem Ballonabschnitt des Katheters aufgenommenen und im wesentlichen axialfest gehaltenen Endoprothese, die radial aufweitbar und nach bestimmungsgemäßer Plazierung bei kollabiertem Ballonabschnitt vom Katheter trennbar ist. Insbesondere bezieht sich die Erfindung auf einen Katheter zum Eröffnen oder Erweitern von Gefäßen.

Eine Vorrichtung der vorgenannten Art und Zweckbestimmung ist aus der EP-A-0 183 372 vorbekannt, bei dem auf einem mit Druckmedium beaufschlagbaren und dadurch radial aufweitbaren Ballonabschnitt eines Katheters eine gleichfalls radial aufweitbare und danach vom Katheter trennbare Endoprothese aufgenommen ist.

Bei der Endoprothese der vorbekannten Vorrichtung handelt es sich um ein Gewebe aus im Winkel zur Prothesenlängserstreckung verlaufenden und einander kreuzenden Kunststofffasern mit in Längsrichtung eingewobenen Kettfäden. Derartige Gewebe sind unproblematisch aufweitbar, erfahren dabei aber eine dem Grad der jeweiligen Aufweitung entsprechende Verkürzung. Dies hat einerseits zur Folge, daß die längenunveränderlichen Kettfäden im implantierten Zustand der Endoprothese über deren stirnseitige Enden vorstehen. Dadurch kann es zu Gefäßverletzungen und erneuten Gefäßverschlüssen kommen. Insbesondere in gekrümmten Gefäßen würden die überstehenden und geradeaus gerichteten Kettfäden in das Gefäßlumen hineinragen, wodurch es zu Störungen im Blutfluß mit Thrombose und Embolie kommen könnte.

Andererseits sind die beim Aufweiten in einem Gefäß eintretenden Verkürzungen nur schwer vorherbestimmbar. Dadurch kann es bei Implantaten im Bereich von Gefäßabzweigungen, etwa von der Hauptschlagader, zu unerwünschten Überständen der expandierten Prothese in die Hauptschlagader kommen, oder eine zu behandelnde Gefäßverengung wird von einer implantierten Prothese nicht auf der erforderlichen Länge abgedeckt. Ferner haben diese schlauchförmigen Gewebe als Endoprothese nur ein geringes Beharrungsvermögen in der Aufweitlage, so daß äußere Einwirkungen nach Implantation leicht zu Deformationen und damit zu höchst unerwünschten Querschnittsreduzierungen bis hin zu erneuten Gefäßverschlüssen führen können. Dies darum, weil derartige Gewebe nicht elastisch sind.

Aus der EP-A-0 221 570 ist eine ganz ähnliche Endoprothese in Form eines gitterartigen zylindrischen Gebildes aus einander kreuzenden Drähten vorbekannt, die vorzugsweise in den Kreuzungspunkten durch Schweißen, Löten oder Kleben fest miteinander verbunden sind, alternativ dazu aber auch in den Kreuzungspunkten unverbunden belassen sein können.

Bei der vorbekannten Ausführungsform mit in den Kreuzungspunkten fest miteinander verbundenen Drähten handelt es sich um ein starres System. Zur Implantation in primär gekrümmten Gefäßen und in solchen Bereichen, in denen die Endoprothese Bewegungen unterworfen ist, wie beispielsweise im Bereich einer Armbeuge, sind starre Systeme nicht geeignet, weil sie gekrümmten Gefäßverläufen und Beugebewegungen nicht folgen können. Angesichts der Starrheit dieser Endoprothese treten an den Übergängen von der Prothese zu nicht behandelten Gefäßbereichen große mechanische Beanspruchungen der nicht behandelten Gefäßsegmente auf und es wird zum einen die Gefäßpulsation gestört und zum anderen entsteht bei Krümmung eines derartigen Gefäßes eine übermäßige Gefäßbelastung. Außerdem erfahren so ausgebildete Endoprothesen beim Aufweiten eine Verkürzung mit den oben bereits dargelegten unerwünschten Folgen.

Wenn hingegen die einander kreuzenden Einzeldrähte in den Kreuzungspunkten nicht miteinander verbunden sind, liegen einfache schlauchförmige Drahtgewebe vor, die beim Aufweiten eine extreme Verkürzung erfahren, aber keinerlei Beharrungsvermögen in der Aufweitlage haben. Mit Endoprothesen aus derartigen Drahtgeweben können Gefäßverschlüsse nicht dauerhaft eröffnet werden, weil solche Prothesen sich unter der Wirkung der von den Gefäßwänden ausgehenden radialen Rückstellkräfte bei gleichzeitiger Verlängerung in ihre Ursprungsform zurückbilden würden. Erneute Gefäßverschlüsse wären die unvermeidbare Folge, wobei das Drahtgewebe der Endoprothese Thrombenbildungen begünstigt.

Schließlich sind aus der FR-A-2 525 896 auch schon Endoprothesen in Form schlauchartiger Gebilde aus einander kreuzenden Einzeldrähten bekannt, wobei die Einzeldrähte Federdrähte mit Vorkrümmung sind. Die vorbekannte Endoprothese wird im radial deformierten Zustand in ein Gefäß eingeführt und weitet sich nach der Implantation infolge Freigabe der Federvorspannung auf.

Naturgemäß erfahren derartige Endoprothesen beim Aufweiten ebenfalls eine axiale Verkürzung mit allen daraus resultierenden negativen Folgen. Da Radius und Länge bei dieser Prothese in einem konstanten Verhältnis stehen, tritt bei einer Verringerung des Durchmessers, die durch äußere Kompression an einer lokalisierten Stelle verursacht sein kann, notwendig eine Verlängerung der Prothese ein. Dadurch werden große Scherkräfte der einige Zeit nach Implantation eingewachsenen Prothesendrähte auf die Gefäßwand ausgeübt. Dies kann zu Gefäßwandirritationen und mithin zur Thrombenbildung und nachfolgendem Gefäßverschluß oder zumindest einer Gefäßverengung führen.

Obgleich es sich nicht um ausgesprochen starre Systeme handelt, sind auch derartige Endoprothesen allenfalls bedingt zur Implantation in primär gekrümmten Gefäßen oder in Beugungsbereichen geeignet. Der entscheidende Nachteil aber besteht darin, daß derartige Endoprothesen im implantierten Zustand unter radialer Spannung an den Gefäßwänden anliegen und daß dadurch letztere geschädigt werden können.

Diesen Unzulänglichkeiten hilft die Erfindung dadurch ab, daß die im Oberbegriff des Patentanspruchs 1 angegebene Endoprothese als durch Stricken, Häkeln oder durch sonstige Arten der Maschenbildung hergestellter aus Metall- oder Kunststoff-Fadenmaterial jeweils guter Gewebeverträglichkeit aufgebauter, radial aufweitbarer und danach in der Aufweitlage verharrender flexibler Schlauch ausgebildet ist.

Unter Stricken, Häkeln oder dergleichen sind im Sinne der vorliegenden Erfindung Maschenbildungen jedweder Art zu verstehen, bei denen jeweils einander umschlingende Bogenstücke verschiedener Einzelfasern ohne feste Verbindung in den jeweiligen Kreuzungspunkten die Maschen bilden. Bei der erfindungsgemäßen Endoprothese liegt somit eine begrenzte Bewegbarkeit der einander umschlingenden Bogenstücke der Einzelfasern gegeneinander vor mit der Folge, daß beim Aufweiten eines derartigen "Gestrickes" die einzelnen Bogenstücke plastische Verformungen erfahren und dadurch derartige Endoprothesen in ihren Aufweitlagen verharren.

Schlauchförmige Gestricke sind beispielsweise beim Aufbau von Filtern bekannt, haben aber als radial aufweitbare und dann in ihren Aufweitlagen verharrende Endoprothesen ersichtlich noch keine Verwendung gefunden. Eingehende Versuche haben indessen die besondere Brauchbarkeit gestrickter, oder gehäkelter oder in sonstiger Weise durch Maschenbildung hergestellter Endoprothesen erwiesen. Derartige Endoprothesen sind im nicht aufgeweiteten und aufgeweiteten Zustand flexibel. Sie können daher in einfacher Weise im nicht aufgeweiteten Zustand selbst durch gekrümmte Gefäße geführt und in diesen auch aufgeweitet werden. Sie zeichnen sich aber auch im aufgeweiteten Zustand durch eine durchaus erwünschte Elastizität bei gutem Beharrungsvermögen aus und vermögen sich daher den Gegebenheiten der Gefäße anzupassen.

Derartige Implantate kehren angesichts der ihnen eigenen Federwirkung nach begrenzten Deformationen, etwa infolge äußerer Krafteinwirkungen, wieder in ihre nach dem radialen Aufweiten eingenommene Stellung zurück, so daß die Gefahr erneuter Verschlüsse im Bereich derartiger Implantate zumindest wesentlich reduziert ist.

Ein weiterer wichtiger Vorteil einer so ausgebildeten Endoprothese besteht darin, daß diese innerhalb vorbestimmter Grenzen stufenlos radial aufweitbar und somit problemlos an die individuellen Gegebenheiten des jeweiligen Patienten anpaßbar ist. Insbesondere ist dies der Fall, wenn gemäß einer Weiterbildung der Erfindung die Endoprothese vor deren bestimmungsgemäßer Verwendung in solcher Weise radial verformt und im Durchmesser reduziert worden ist, daß die einzelnen Maschen mit Spiel ineinandergreifen.

Gemäß einer zweckmäßigen Ausgestaltung der Erfindung kann die als radial aufweitbarer Schlauch ausgebildete Endoprothese aus wenigstens zwei einander umschließenden durch Stricken, Häkeln od.dgl. hergestellten Lagen bestehen, die durchaus auch unterschiedlich ausgeführt sein können. Demgemäß kann in Abhängigkeit von der Wahl der Anzahl der Lagen die Widerstandsfähigkeit derartiger Endoprothesen gegen Deformationen infolge äußerer Krafteinwirkungen dem jeweiligen Bedarfsfall entsprechend ausgewählt werden.

Die zuletzt genannte Ausgestaltung kann auch durch Verankerungsmittel gekennzeichnet sein, die beim radialen Aufweiten der Endoprothese deren äußere Lage durchdringen, und die Verankerungsmittel können mit einer inneren Lage verbunden sein sowie sich radial von letzterer forterstreckende Ankerstifte aufweisen, die sich in der Aufweitlage durch die äußere Lage hindurcherstrecken und in eine Gefäßwand oder das einen Körperhohlraum umgebende Gewebe eingreifen.

Der die Endoprothese bildende Schlauch kann aber auch aus Fadenmaterial hergestellt sein, das seinerseits aus wenigstens zwei jeweils zu einem Strang zusammengefaßten und gemeinsamen durch Stricken, Häkeln oder sonstige Schlingenbildung verarbeiteten Einzelfäden besteht. So hergestellte Endoprothesen zeichnen sich durch große Widerstandsfähigkeit gegen Deformationen infolge äußerer Krafteinwirkungen aus.

Eine andere Weiterbildung ist durch eine plastische Verformung des den flexiblen Schlauch bildenden Fadenmaterials im Bereich der Maschen beim radialen Aufweiten der Endoprothese gekennzeichnet.

Es kann sich auch, ebenfalls gemäß einer Weiterbildung, wenigstens bei einem Teil des Fadenmaterials, aus dem der flexible Schlauch hergestellt ist, um ein mit Lochungen oder Perforationen versehenes Hohlmaterial z.B. zur kontinuierlichen Medikamentenabgabe handeln.

Nach einer anderen Weiterbildung kann das Fadenmaterial, aus dem die Endoprothese hergestellt ist, mit einer Beschichtung aus gewebeverträglichem Material versehen sein. Der durch Stricken, Häkeln od.dgl. aus Fadenmaterial hergestellte flexible Schlauch kann aber auch außen- und/oder innenseitig mit einer manschettenartigen Umhüllung aus dehnbarem, aber flüssigkeitsdichtem Material, wie Latex, versehen sein.

Eine gleichfalls wichtige Ausgestaltung der Erfindung ist gekennzeichnet durch die Ausbildung der Endoprothese als Gefäßklappe mit sich beidendig axial über den durch Stricken, Häkeln oder auf ähnliche Weise hergestellten Schlauch hinauserstreckenden schlauchförmigen Abschnitten, die an ihren Enden mit nicht aufweitbaren Halteringen versehen sind und von denen der eine Abschnitt mit einer flüssigkeitsdichten Auskleidung oder Beschichtung aus Latex oder einem anderen geeigneten Material versehen ist. Beim Implantieren einer derartigen Gefäßklappe können die nicht aufweitbaren Halteringe am radialen Aufweitvorgang nicht teilnehmen, so daß sich beidendig des schlauchförmigen Mittelteils Haltekörbe ausbilden, von denen der mit einer flüssigkeitsdichten Auskleidung oder Beschichtung versehene Haltekorb zusammen mit einer nachträglich noch in die implantierte Gefäßklappe einzubringenden Ventilkugel einen wahlweisen dichten Abschluß vermittelt, während im Bereich des anderen Endabschnittes selbst beim Anliegen der Ventilkugel am Haltering mangels einer flüssigkeitsdichten Beschichtung oder Auskleidung keine Absperrung eintreten kann.

Eine andere vorteilhafte Ausgestaltung der Erfindung besteht darin, daß die Endoprothese als Taschenklappe, etwa als Aortenklappe, mit wenigstens zwei an einem Ende eines durch Stricken, Häkeln oder auf ähnliche Weise hergestellten Schlauchteils angeordneten rautenförmigen Klappen ausgebildet ist, wobei die Klappen aus feinmaschigem Gewebe bestehen, dessen miteinander verwebte Fasern jeweils zu zwei Rautenkanten etwa parallel verlaufen und die Klappen in dreiecksförmigen Ausschnitten des Schlauchteils mit jeweils einer etwa in Axialrichtung des Schlauchteils verlaufenden Diagonalen aufgenommen, sowie längs zweier Rautenkanten mit den Kanten der dreiecksförmigen Ausschnitte im Schlauchteil verbunden sind, etwa durch Vernähen oder Häkeln mittels Federdraht.

Eine derartige Taschenklappe ist beispielsweise dergestalt in Herzkammern einsetzbar, daß die in dreiecksförmige Ausschnitte des Schlauchteils eingesetzten Klappen im Bereich der Abgänge der Herzkranzarterien liegen, während das Schlauchteil sich in die Herzkammer hineinerstreckt und dort durch radiales Aufweiten lagerichtig implantiert sowie im Bedarfsfalle durch geeignete Verankerungsmittel fixiert ist. Die Klappen dieser Endoprothese bestehen aus feinmaschigem Gewebe, dessen Lücken im implantierten Zustand durch Blutgerinsel verkleben oder aber auch durch Silikon abgedichtet sein können.

Die zuletzt erörterte Endoprothese kann aber auch umgekehrt implantiert werden, so daß sich das Schlauchteil dann in der Aorta befindet und nicht in den Ventrikel reicht. Da das durch Stricken, Häkeln oder auf ähnliche Weise hergestellte Schlauchteil an seinem klappenseitigen Ende dreiecksförmig ausgeschnitten ist, muß die Implantation so erfolgen, daß die Abgänge der Herzkranzarterien frei sind. Der Verlust an Elastizität kann in einfacher Weise durch einen die Naht zwischen den Klappen und dem Schlauchteil bildenden Federdraht ausgeglichen werden, der im implantierten Zustand das Schlauchteil und die Klappen zusätzlich an die Gefäßwand andrückt. Die Klappen sind im übrigen an ihren Spitzen abgerundet und an den - im implantierten Zustand - der Aortenwand nahen Rändern miteinander verbunden.

Im Interesse einer erleichterten Einführbarkeit sieht eine andere wichtige Ausgestaltung vor, daß zur Vermeidung einer Stufe zwischen der Katheterspitze und dem Ballonabschnitt ein zu letzterem hin leicht ansteigender Konus vorgesehen ist, der gleichzeitig zur Katheterspitze hin eine axiale Begrenzung für die im nicht aufgeweiteten Zustand auf dem Ballonabschnitt aufgenommene Endoprothese bildet. Dadurch wird die bei derartigen Kathetern ansonsten anzutreffende Stufe im Bereich zwischen der Katheterspitze und dem Ballonabschnitt vermieden und mithin das Einführen des Katheters durch eine Hautpunktionsstelle oder das Vorschieben in einem Gefäß wesentlich erleichtert.

An den genannten Konus kann sich auch ein zum Ballonabschnitt hin abfallender Konus anschließen, der eine Begrenzung für die Einführhülse bildet, welche die auf dem Ballonabschnitt aufgenommene Endoprothese umschließt. Ein zum Ballonabschnitt des Katheters abfallender Konus reduziert auch die Gefahr des Hängenbleibens eines aufgedehnten Implantats im Bereich einer Krümmung, wenn der Katheter zurückgezogen wird.

Anhand der beigefügten Zeichnung sollen nachstehend ein Ballonkatheter mit einer auf dem Ballonabschnitt aufgenommenen, radial aufweitbaren und in der Aufweitlage fixierbaren Endoprothese, eine Ausführungsmöglichkeit einer derartigen Endoprothese sowie das Eröffnen eines durch einen Thrombus verschlossenen Gefäßes und im Zusammenhang damit die Implantation einer Endoprothese im Bereich des Thrombus im Gefäß erläutert werden. In schematischen Ansichten zeigen:

Fig. 1 einen Katheter mit einem radial aufweitbaren Ballonabschnitt und einer auf diesem axialfest aufgenommenen, in einer vorbestimmten Aufweitlage fixierbaren Endoprothese,

Fig. 2 einen Katheter wie in Fig. 1, jedoch mit einem aufgeschobenen, teilweise im Längsschnitt gezeigten Schlauch, an dem sich die auf dem Ballonabschnitt aufgenommene Endoprothese auf der von der Einführrichtung abgewandten Seite abstützt, und mit einer dünnwandigen, die Endoprothese bis zu dem von der Katheterspitze zugewandten Ende überdeckenden Hülle, die den aufgeschobenen Schlauch umschließend sich auf der von der Katheterspitze entfernten Seite der Endoprothese forterstreckt,

Fig. 3 in einem Längsschnitt gemäß der Schnittlinie III-III in Fig. 1 die radial aufgeweitete und in der Aufweitlage fixierte Endoprothese sowie den Katheter mit dem ebenfalls aufgeweiteten, teilwiese geschnitten dargestellten Ballonabschnitt,

Fig. 4 in einer Ansicht wie in Fig. 2 einen abgewandelten Katheter mit einem Doppelkonus im Bereich der Katheterspitze und mit Distanzmitteln auf dem sich vom Ballonabschnitt nach der von der Spitze abgewandten Seite forterstreckenden Bereich,

Fig. 5 eine als schlauchartiges Drahtgestricke ausgebildete Endoprothese für sich allein vor dem radialen Aufweiten,

Fig. 6 einen Querschnitt durch die Endoprothese nach Fig. 4, und zwar in vollen Linien im nicht aufgeweiteten Zustand und in strichpunktierten Linien nach dem Aufweiten,

Fig. 7 in einem vergrößerten Ausschnitt gemäß VII. in Fig. 6 die Ausbildung der Maschen des Drahtgestrickes in der Ursprungslage vor dem radialen Aufweiten und in der Aufweitlage.

Fig. 8 in einer Längsschnittansicht einen vergrößerten Ausschnitt aus einer nicht aufgeweiteten Endoprothese mit zwei einander umschließenden Gestrickelagen und einem an der inneren Gestrickelage befestigten Verankerungsmittel, das einen sich im Ringspalt zwischen den Gestrickelagen radial nach außen erstreckenden Ankerstift besitzt,

Fig. 9 in einer Ansicht wie in Fig. 8 die axiale Fixation der Endoprothese infolge Eingriffs der durch die äußere Gestrickelage hindurchgedrungenen und in die Gefäßwandung eingreifenden Ankerstifte,

Fig. 10 eine als Gefäßklappe ausgebildete Endoprothese für sich allein vor dem radialen Aufweiten in einer Längsschnittansicht,

Fig. 11 Die Gefäßklappe nach Fig. 10 im implantierten Zustand innerhalb eines nur angedeuteten Gefäßes, ebenfalls in einer Längsschnittansicht,

Fig. 12 eine als Aortenklappe ausgebildete Gefäßklappe für sich allein in nicht aufgeweitetem Zustand,

Fig. 13 die Aortenklappe nach Fig. 12 im radial aufgeweitetem Zustand,

Fig. 14 eine im Bereich der Abgänge der Herzkranzarterien implantierte Aortenklappe,

Fig. 15 ein durch einen Thrombus verschlossenes Gefäß mit einem eingeführten Führungsdraht in einem Längsschnitt durch das Gefäß,

Fig. 16 in einer Ansicht wie in Fig. 15, das Gefäß nach durchgeführter Ballonkatheter-Kanalisation mit dem dann komprimierten Thrombus,

Fig. 17 in einer Ansicht wie in Fig. 15 einen in das Gefäß eingeführten Ballonkatheter mit einer auf dem Ballonabschnitt aufgenommenen Endoprothese im Bereich des komprimierten Thrombus vor der radialen Aufweitung des Ballonabschnittes und der Endoprothese,

Fig. 18 gleichfalls in einer Ansicht wie in den Fig.15 bis 17 die durch radiale Aufweitung des Ballonabschnittes des Katheters infolge Druckmedienbeaufschlagung an die Gefäßwand komprimierte Endoprothese und

Fig. 19 ebenfalls in einer Längsschnittansicht durch das Gefäß die in diesem im Bereich des komprimierten Thrombus implantierte Endoprothese nach dem Entfernen des Ballonkatheters und des in das Gefäß eingebrachten Führungsdrahtes.

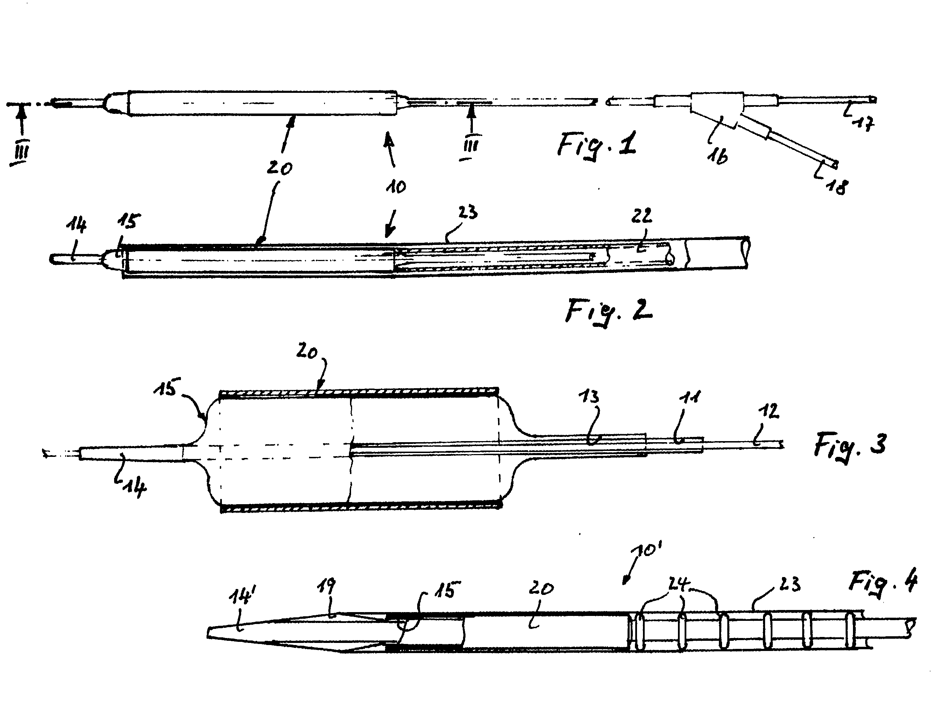

Bei dem in den Fig. 1 bis 4 schematisch veranschaulichten Katheter 10, 10' handelt es sich um einen bekannten doppellumigen Katheter, dessen zentrales Lumen 11 sich axial durch den Katheter hindurcherstreckt und zum Aufschieben des Katheters auf einen Führungsdraht 12 dient, wie Fig. 3 zeigt. Ein das erste Lumen konzentrisch umschließendes zweites Lumen 13 mündet in einem in der Nähe der Katheterspitze 14 angeordneten Ballonabschnitt 15 aus. Die annähernd über die gesamte Axialerstreckung des Katheters koaxial geführten Lumen 11, 13 teilen sich in einem endseitigen Winkelstück 16, was indessen hier nur insoweit interessiert, als das zentrale Lumen 11 zum Aufschieben auf den Führungsdraht 12 mit der sich endseitig von dem Winkelstück forterstreckenden Leitung 17 fluchtet, während das in dem Ballonabschnitt 15 ausmündende Lumen 13 mit der im Winkel dazu verlaufenden Leitung 18 in Strömungsverbindung steht.

Im Unterschied zu vorbekannten Ballonkathetern ist bei der Erfindung auf dem infolge Druckmedienbeaufschlagung radial aufweitbaren Ballonabschnitt 15 des Katheters eine Endoprothese 20 im wesentlichen axialfest aufgenommen, die in unten noch zu beschreibender Weise infolge radialer Aufweitung zum Beispiel in einem Gefäß implantierbar und vom Katheter trennbar ist.

Die Endoprothese kann dabei unter radialer Vorspannung auf dem Katheter 10 aufgenommen und dadurch auf diesem axial festgelegt sowie infolge radialer Aufweitung und Arretierung in der Aufweitlage vom Katheter trennbar sein.

Anstelle der axialen Fixation der Endoprothese auf dem Ballonabschnitt 15 des Katheters 10 infolge radialer Vorspannung oder zusätzlich zu letzterer kann sich die Endoprothese 20 auch auf der von der Einführrichtung entfernten Seite an einem zuvor auf den Katheter aufgeschobenen Schlauch 22 abstützen. Dies zeigt Fig. 2.

Ferner zeigt Fig. 2 eine die Endoprothese 20 über ihre gesamte Länge konzentrisch umschließende Hülle 23, die sich auf der von der Katheterspitze 14 entfernten Seite forterstreckt und die Endoprothese beim Einführen des Katheters 10 vor Beschädigungen durch die Haut, Muskulatur oder Gefäßwände sowie gegen Verrutschen auf dem Katheter schützt. Die schlauchartige Hülle, die aus dünnwandigem Kunststoff einer gewissen Formfestigkeit besteht, vermag sich etwaigen Krümmungen des Einführweges problemlos anzupassen und wird nach der lagerichtigen Plazierung der Endoprothese von dieser nach der von der Katheterspitze entfernten Seite zurückgezogen, wobei der die Endoprothese abstützende Schlauch, dessen entferntes Ende die Hülle mindestens um das Längenmaß der Endoprothese überragt, in seiner aus Fig. 2 ersichtlichen Abstützlage verbleibt.

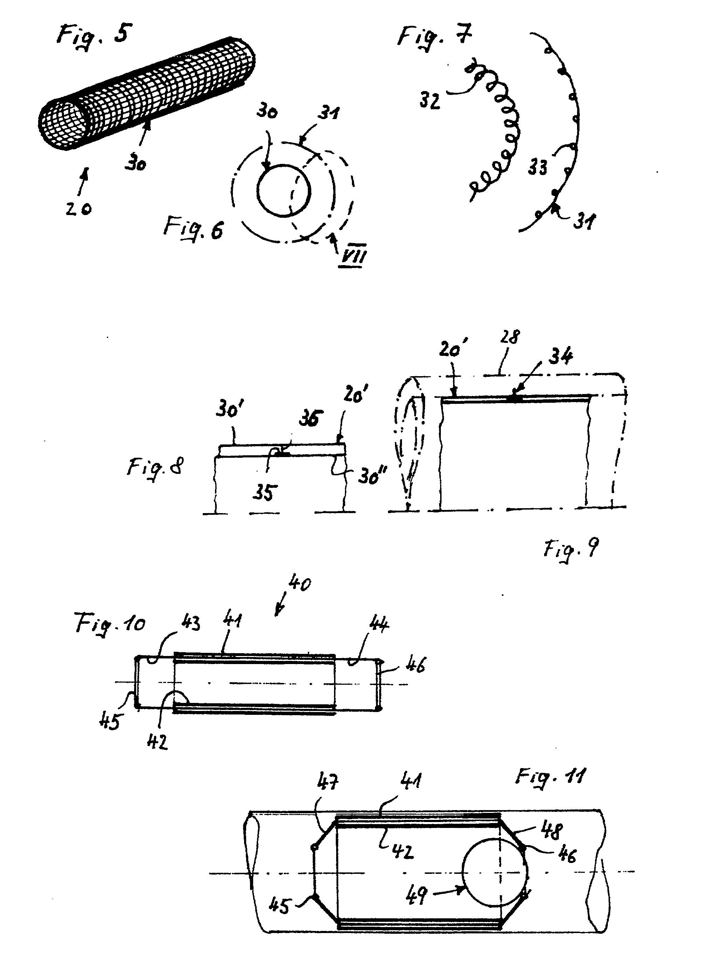

Bei dem in Fig. 4 veranschaulichten Katheter 10' ist zwischen der Katheterspitze 14' und dem Ballonabschnitt 15 mit der darauf aufgenommenen Endoprothese 20 ein Doppelkonus 19 angeordnet. Dadurch ist die bei dem Katheter 10 nach den Fig. 1 bis 3 zwischen Spitze und Ballonabschnitt auftretende Stufe vermieden und das Einführen in das Gefäß erleichtert. Auf der vom Ballonabschnitt entfernten Seite ist der Katheter mit in Katheterlängsrichtung in Abständen voneinander angeordneten Distanzringen 24 versehen, an denen sich die in gleicher Weise wie bei dem Katheter nach Fig. 2 aufgeschobene und bis über die Endoprothese reichende Hülle 23 radial abstützt. Ferner sichert der dem Ballonabschnitt benachbarte Ring 24 die Endoprothese in ihrer axialen Lage auf dem Ballonabschnitt.

Ballonkatheter der erwähnten Art dienen zum Eröffnen und/oder Erweitern beispielsweise von durch Thromben verschlossenen oder verengten Gefäßen. Fig. 15 zeigt schematisch ein Gefäß 26, das durch einen Thrombus 25 verschlossen ist. Um ein derart verschlossenes -oder auch nur verengtes- Gefäß zu eröffnen, muß zunächst in bekannter Weise ein Führungsdraht 12 in das Gefäß eingeführt werden, worauf ein Ballonkatheter mit seinem einen Lumen über den Führungsdraht 12 in dem Gefäß in solcher Wiese vorgeschoben wird, daß der infolge Druckmedienzufuhr durch das andere Lumen radial aufweitbare Ballonabschnitt 15 im Bereich des Thrombus liegt. Durch radiales Aufweiten des Ballonabschnittes infolge dessen Druckmedienbeaufschlagung über das eine Lumen wird der Thrombus 25 radial komprimiert, was zu der in Fig. 16 schematisch veranschaulichten Aufweitung 27 der Gefäßwand 28 im Bereich des komprimierten Thrombus führt.

Bei der bekannten Ballon-Rekanalisation wird nunmehr nach Wegnahme der die radiale Aufweitung des Ballonabschnittes bewirkenden Druckmedienbeaufschlagung der Katheter zusammen mit dem Führungsdraht aus dem Gefäß zurückgezogen. Es kann somit, jedenfalls im Laufe der Zeit, zu erneuten Gefäßverengungen oder gar zu Reverschlüssen kommen. Demgemäß eröffnet der durch die Erfindung vorgeschlagene Katheter die Möglichkeit, im Bereich des komprimierten Thrombus eine einen erneuten Gefäßverschluß bzw. eine erneute Gefäßverengung verhindernde Endoprothese 20 zu implantieren.

Demgemäß wird nach der vorstehend erläuterten Ballonrekanalisation nach Wegnahme der Druckmedienbeaufschlagung des Ballonabschnittes der Katheter unter Belassung des Führungsdrahtes 12 aus dem Gefäß 26 zurückgezogen und ein Katheter nach der Erfindung, der eine radial aufweitbare und in einer vorbestimmten Aufweitlage arretierbare Endoprothese 20 auf seinem Ballonabschnitt 15 aufnimmt, auf den Führungsdraht 12 aufgeschoben, bis der Ballonabschnitt mit der darauf aufgenommenen Endoprothese in den Bereich des komprimierten Thrombus 25 im Gefäß gelangt. Dies zeigt Fig. 17.

Nachdem die auf dem Ballonabschnitt 15 des Katheters aufgenommene Endoprothese 20 lagerichtig im Gefäß 26 plaziert ist, wird die Endoprothese durch Druckmedienbeaufschlagung des Ballonabschnittes 15 des Katheters radial aufgeweitet und innenseitig an die Gefäßwand 28 komprimiert, wobei der bereits vorkomprimierte Thrombus 25 eine weitere Kompression und die umgebende Gefäßwand eine entsprechende zusätzliche radiale Ausdehnung erfahren. In der aus Fig. 18 ersichtlichen radialen Aufweitlage erfährt die Endoprothese eine Fixation, was unten noch näher erläutert wird.

Nach der Fixation der Endoprothese in ihrer an die Wand komprimierten Aufweitlage wird der Ballonabschnitt 15 des Katheters infolge Wegnahme der Druckmedienbeaufschlagung radial reduziert und danach der Katheter samt Führungsdraht 12 aus dem Gefäß 26 zurückgezogen. Dabei verbleibt die Endoprothese 20 als Implantat im Gefäß und verhindert eine mögliche Rückbildung des komprimierten Thrombus 25 sowie die dadurch begründete Gefahr erneuter Gefäßverengungen oder Gefäßverschlüsse.

Mögliche Ausführungsformen der auf dem Ballonabschnitt 15 des Katheters 10 axialfest gehaltenen und infolge radialer Aufweitung des Ballonabschnittes ebenfalls radial aufweitbaren und in einer vorbestimmten Aufweitlage gegen nachfolgende Querschnittsreduzierungen arretierbaren Endoprothese veranschaulichen die Fig. 5 bis 11.

Bei der in den Fig. 5 bis 9 veranschaulichten Ausführungsform der Endoprothese 20 handelt es sich um ein schlauchförmiges Gestricke 30 aus Metalldrähten oder Kunststoffasern jeweils guter Gewebeverträglichkeit im Durchmesserbereich von etwa 0,1 mm, die durch eng anliegendes Umstricken eines (nicht dargestellten) Kerns hergestellt wird, der danach entfernbar ist. Die Querschnittsansicht nach

Fig. 6 zeigt in vollen Linien die Endoprothese nach Fig. 5 im Ursprungszustand, also vor dem radialen Aufweiten, während die Aufweitlage bei 31 in strickpunkterten Linien angedeutet ist.

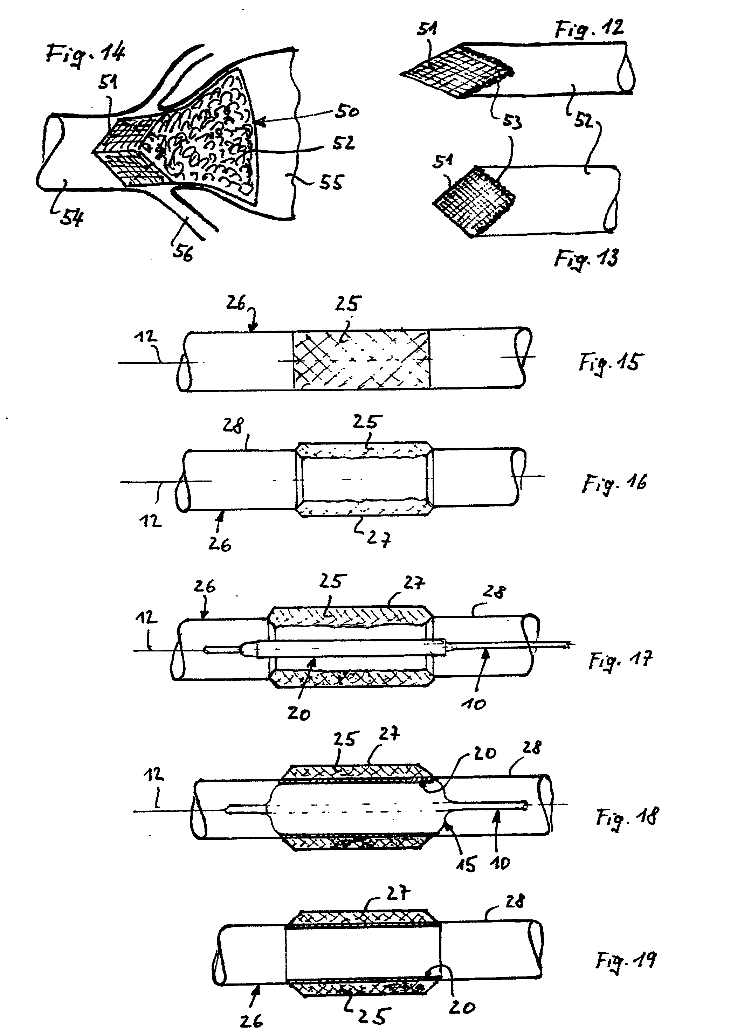

Bei dem die Endoprothese 20 bildenden schlauchförmigen Gestricke 30 handelt es sich um ein in der Ursprungslage, also vor dem radialen Aufweiten, lockeres Gestricke, dessen einzelne Maschen 32 locker, also mit gegenseitigem Spiel, ineinandergreifen und etwa die in Fig. 7 angedeutete Schlingenform aufweisen. Das lockere Ineinandergreifen der einzelnen Maschen kann im Bedarfsfalle durch Zusammendrücken des Gestrickes und eine damit einhergehende Durchmesserreduzierung vor dessen Verwendung als Endoprothese verbessert werden. Beim radialen Aufweiten des Gestrickes findet eine Verformung der die einzelne Maschne bildenden Schlingen über den elastischen Bereich des Fadenmaterials hinaus statt, mithin also eine plastische Verformung des Fadenmaterials, bis diese eine etwa der Darstellung in Fig. 7 entsprechende, bei 33 angedeutete Gestalt angenommen haben.

Angesichts der beim radialen Aufweiten des schlauchförmigen Gestrickes eintretenden plastischen Verformung des die Maschen bildenden Fadenmaterials findet eine selbsttätige Fixation in der jeweiligen Aufweitlage statt, ohne daß es dafür irgendwelcher zusätzlicher Maßnahmen bedarf. Demgemäß ist innerhalb vorbestimmter Grenzen die als schlauchförmiges Gestricke ausgebildete Endoprothese stufenlos aufweitbar und daher den Bedürfnissen des jeweiligen Anwendungsfalles weitgehend anpaßbar.

Anstelle schlauchförmiger Gestricke können auch durch Häkeln, Weben, Knüpfen oder sonstige Weise aus Fadenmaterial hergestellte Endoprothesen eingesetzt werden.

Bei der schematisch in den Fig. 8 und 9 gezeigten Ausführungsform besteht die Endoprothese 20' aus zwei einander umschließenden Gestrickelagen 30', 30'', zwischen denen Verankerungsmittel 34 ausgenommen sind, die einen an der inneren Gestrickelage 30'' befestigten Druckteller 35 und einen sich davon radial nach außen forterstreckenden Ankerstift 36 besitzen. Wie Fig. 8 zeigt, erstrecken sich die Ankerstifte vor dem radialen Aufweiten der Endoprothese in einem Ringspalt zwischen den Gestrickelagen 30', 30'', durchdringen aber beim radialen Aufweiten die äußere Gestrickelage 30' und greifen in der aus Fig. 8 ersichtlichen Weise in die in dieser Figur strichpunktiert angedeutete Gefäßwand 28 ein. Dadurch ist eine formschlüssige Fixation der Endoprothese im Gefäß verwirklicht.

Bei der in den Fig. 10 und 11 veranschaulichten Endoprothese handelt es sich um eine Gefäßklappe 40 mit einem aus zwei einander umschließenden Gestrickelagen 41, 42 bestehenden schlauchförmigen Mittelteil und zwei schlauchförmigen Abschnitten 43, 44 aus aufweitbarem Material, die sich beidseitig axial über die Gestrickelagen hinauserstrecken und beidendig mit nicht aufweitbaren Halteringen 45, 46 fest verbunden sind. Bei den Abschnitten 43, 44 kann es sich ebenfalls um Gestrickelagen handeln, wobei einer dieser Abschnitte, etwa der Abschnitt 44, mit einer flüssigkeitsdichten Auskleidung oder Beschichtung versehen ist, etwa aus Latex.

Die Implantation der Gefäßklappe erfolgt in der oben in Verbindung mit der Endoprothese 20 erläuterten Weise, wobei der Ballonabschnitt des einzusetzenden Katheters die axiale Länge der Gestrickelagen 41, 42 nicht überschreiten und über letztere auf keiner Seite vorstehen darf. Wenn nach lagerichtiger Plazierung in einem Gefäß die Gefäßklappe infolge Druckmedienbeaufschlagung des Ballonabschnittes des Katheters radial aufgeweitet wird, nehmen die beidendigen Halteringe 45, 46 der axial vorstehenden Abschnitte 43, 44 an der Aufweitung nicht teil, so daß sich beidendig von den Gestrickelagen ein proximaler und ein distaler Haltekorb 47, 48 ausbilden, wie dies Fig. 11 zeigt.

Nach der Implantation der Gefäßklappe und dem Zurückziehen des Katheters aus dem Gefäß wird mittels eines bekannten Katheters eine mit einem ablösbaren Schlauch versehene Kugelhülle in das Gefäß eingebracht und durch einen der Halteringe hindurch in die implantierte Gefäßklappe 40 eingeführt sowie anschließend durch Zufuhr von Silicon oder einem ähnlichen Material innerhalb der Gefäßklappe zur Kugelform aufgeweitet, um nach dem Ablösen der Zuleitung dann eine Ventilkugel 49 der Gefäßklappe zu bilden. Angesichts der Auskleidung oder Beschichtung des den Haltekorb 48 bildenden Abschnittes 44 vermittelt die Ventilkugel 49 im Zusammenwirken mit dem Haltekorb 48 einen dichten Gefäßabschluß. Im Bereich des Haltekorbs 47 kann hingegen auch beim Anliegen der Ventilkugel 49 kein Gefäßabschluß auftreten, weil der durch den Haltering 45 abgeschlossene Abschnitt 43 mit keiner Auskleidung oder Beschichtung versehen und daher auch beim Anliegen der Ventilkugel am Haltering 45 nicht absperrbar ist.

Bei der in den Fig. 12 bis 14 gezeigten Endoprothese handelt es sich um eine Aortenklappe 50 mit drei rautenförmigen Klappen 51, die an einem Ende eines durch Häkeln, Stricken oder auf ähnliche Weise hergestellten Schlauchteils 52 angeordnet und jeweils mit in Axialrichtung des Schlauchteils verlaufenden Diagonalen längs zweier Rautenkanten an den Kanten dreiecksförmiger Ausschnitte im Schlauchteil durch Vernähen oder Verhäkeln verbunden sind, wie dies in den Fig. 12 und 13 bei 53 angedeutet ist.

In Fig. 12 ist eine Aortenklappe für sich allein vor dem radialen Aufweiten veranschaulicht, wobei nur eine der drei in dreiecksförmigen Ausschnitten des Schlauchteils 52 aufgenommenen rautenförmigen Klappen 51 sichtbar ist, deren Hauptdiagonalen sich in Längsrichtung des Schlauchteils erstrecken. Die Klappen 51 bestehen zum Beispiel aus feinmaschigem Drahtgewebe, bei dem die einander kreuzenden Drähte jeweils parallel zu zwei Rautenkanten verlaufen. Fig. 13 hingegen veranschaulicht die Aortenklappe 50 im radial aufgeweiteten Zustand, in dem die rautenförmigen und jeweils längs zweier Rautenkanten bei 53 mit den Ausschnittkanten des Schlauchteils 52 eine - von oben gesehen - etwa quadratische Gestalt angenommen haben.

Das durch Häkeln, Stricken oder auf ähnliche Weise hergestellte Schlauchteil ist in gleicher Weise wie die oben erläuterte Endoprothese radial aufweitbar und dann in der jeweiligen Aufweitlage durch plastische Deformation der einander umschließenden Geschlinge fixierbar. Zur lagerichtigen Fixation können zusätzliche Verankerungsmittel vorgesehen sein, wie oben in Verbindung mit den Fig. 8 und 9 erläutert worden ist. Die an das Schlauchteil angeschlossenen rautenförmigen Klappen aus feinmaschigem Gewebe vermögen beim radialen Aufweiten des Schlauchteils letzterem unproblematisch zu folgen, weil die einander kreuzenden Fasern des feinmaschigen Gewebes zu jeweils zwei Rautenkanten parallel verlaufen und die Hauptdiagonale der Raute sich in Längsrichtung des Schlauchteils erstreckt. Angesichts dieser Ausbildung und Anordnung der Klappen gehen diese bei fortschreitendem radialen Aufweiten des Schlauchteils mehr und mehr aus der Rautenform zur Quadratform über, wie die Fig. 12 und 13 zeigen.

Fig. 14 schließlich veranschaulicht eine durch eine Aorta 54 implantierte Aortenklappe 50, deren radial aufgeweitetes Schlauchteil in eine Herzkammer 55 hineinragt und in hier nicht weiter interessierender Weise fixiert ist, während die Klappen 51 sich im Bereich der Abgänge 56 der Herzkranzarterien befinden. Das Implantat ist dabei so fixiert, daß angesichts der dreiecksförmigen Ausschnitte des Schlauchteils an seinem klappenseitigen Ende die Abgänge der Herzkranzarterien frei sind.

## Patentansprüche

1. Vorrichtung zum Herstellen oder Erweitern von Verbindungen zu oder zwischen Körperhohlräumen, mit einem Katheter, der einen infolge Druckmedienbeaufschlagung über ein Lumen radial aufweitbaren Ballonabschnitt besitzt, und mit einer auf dem Ballonabschnitt des Katheters aufgenommenen und im wesentlichen axialfest gehaltenen Endoprothese, die radial aufweitbar und in ihrer Aufweitlage nach bestimmungsgemäßer Plazierung bei kollabiertem Ballonabschnitt vom Katheter trennbar ist, insbesondere zum Eröffnen oder Erweitern von Gefäßen, dadurch gekennzeichnet, daß die Endoprothese (20, 20') ein durch Stricken, Häkeln oder durch sonstige Arten der Maschenbildung hergestellter, aus Metall- oder Kunststoff-Fadenmaterial guter Gewebeverträglichkeit aufgebauter, radial aufweitbarer und danach in der Aufweitlage verharrender flexibler Schlauch (30, 40, 50) ist.

2. Vorrichtung nach Anspruch 1, dadurch gekennzeichnet, daß der flexible Schlauch (30, 40, 50) vor seiner Verwendung als Endoprothese (20, 20') in solcher Weise radial verformt und im Durchmesser reduziert wird, daß die einzelnen Maschen mit Spiel ineinandergreifen.

3. Vorrichtung nach Anspruch 2, dadurch gekennzeichnet, daß die als radial aufweitbarer Schlauch (30, 40, 50) ausgebildete Endoprothese aus wenigstens zwei einander umschließenden, durch Stricken, Häkeln od. dgl. hergestellten, ggf. unterschiedlich ausgeführten Lagen besteht.

4. Vorrichtung nach Anspruch 3, gekennzeichnet durch beim radialen Aufweiten der Endoprothese deren äußere Lage durchdringende Verankerungsmittel (34).

5. Vorrichtung nach Anspruch 4, dadurch gekennzeichnet, daß die Verankerungsmittel ( 34) mit einer inneren Lage der Endoprothese verbunden sind und sich radial von letzterer forterstreckende Ankerstifte (36) aufweisen.

6. Vorrichtung nach einem der Ansprüche 1 bis 5, dadurch gekennzeichnet, daß der die Endoprothese bildende Schlauch (30, 40, 50) aus Fadenmaterial hergestellt ist, das seinerseits aus wenigstens zwei jeweils zu einem Strang zusammengefaßten und gemeinsam durch Stricken, Häkeln od. dgl. verarbeiteten Einzelfäden besteht.

7. Vorrichtung nach einem der Ansprüche 2 bis 6, gekennzeichnet durch eine plastische Verformung des den flexiblen Schlauch (30, 40, 50) bildenden Fadenmaterials im Bereich der Maschen beim radialen Aufweiten der Endoprothese.

8. Vorrichtung nach einem der Ansprüche 1 bis 7, dadurch gekennzeichnet, daß es sich bei wenigstens einem Teil des Fadenmaterials, aus dem der flexible Schlauch (30, 40, 50) hergestellt ist, um ein mit Lochungen oder Perforationen versehenes Hohlmaterial z.B. zur kontinuierlichen Medikamentenabgabe handelt.

9. Vorrichtung nach einem der Ansprüche 1 bis 8, dadurch gekennzeichnet, daß das Fadenmaterial, aus dem der die Endoprothese bildende flexible Schlauch (30, 40, 50) hergestellt ist, mit einer Beschichtung aus gewebeverträglichem Material versehen ist.

10. Vorrichtung nach einem der Ansprüche 1 bis 9, dadurch gekennzeichnet, daß der durch Stricken, Häkeln od. dgl. hergestellte flexible Schlauch (30, 40, 50) außen- und/ oder innenseitig mit einer manschettenartigen Umhüllung aus dehnbarem Material, wie Latex, versehen ist.

11. Vorrichtung nach einem der Ansprüche 1 bis 10, gekennzeichnet durch die Ausbildung der Endoprothese als Gefäßklappe mit sich beidendig axial über den durch Stricken, Häkeln oder auf ähnliche Weise hergestellten Schlauch (40) hinauserstreckenden schlauchförmigen Abschnitten (43, 44), die an ihren Enden mit nicht aufweitbaren Halteringen (45, 46) versehen sind und von denen der eine Abschnitt (44) mit einer flüssigkeitsdichten Auskleidung oder Beschichtung aus Latex oder einem anderen geeigneten Material versehen ist.

12. Vorrichtung nach einem der Ansprüche 1 bis 11, dadurch gekennzeichnet, daß die Endoprothese als Taschenklappe (50), etwa als Aortenklappe, mit wenigstens zwei an einem Ende eines durch Stricken, Häkeln oder auf ähnliche Weise hergestellten Schlauchteils (52) angeordneten rautenförmigen Klappen (51) ausgebildet ist, wobei die Klappen aus feinmaschigem Gewebe bestehen, dessen miteinander verwebte Fasern jeweils zu zwei Rautenkanten etwa parallel verlaufen und die Klappen in dreiecksförmigen Ausschnitten des Schlauchteils mit jeweils einer etwa in Axialrichtung des Schlauchteils verlaufenden Diagonalen aufgenommen sowie längs zweier Rautenkanten mit den Kanten der dreiecksförmigen Ausschnitte im Schlauchteil verbunden sind, etwa durch Vernähen oder Häkeln mittels Federdraht.

13. Vorrichtung nach einem der Ansprüche 1 bis 12, dadurch gekennzeichnet, daß zur Vermeidung einer Stufe zwischen der Katheterspitze und dem Ballonabschnitt (15) ein zu letzterem hin leicht ansteigender Konus (19) vorgesehen ist, der gleichzeitig zur Katheterspitze hin eine axiale Begrenzung für die im nicht aufgeweiteten Zustand auf dem Ballonabschnitt aufgenommene Endoprothese bildet.

14. Vorrichtung nach Anspruch 13, dadurch gekennzeichnet, daß der Konus als Doppelkonus (19) mit einem sich zum Ballonabschnitt (15) hin verjüngenden Teil ausgebildet ist.

## Claims

1. Device for making and dilating connections to or between body cavities by means of a catheter provided with a balloon section radially expandable via a lumen by the admission of pressure media, and by means of an endoprosthesis disposed on said balloon section and held there in an essentially axial position, being radially expandable and separable from the catheter balloon section in its deflated balloon state after having been placed in a predetermined manner, particularly for opening or dilating vessels, wherein said endoprosthesis (20, 20') is a flexible tube (30, 40, 50) formed by knitting, crocheting or other forms of meshes, consisting of metal or plastic filament material of good biocompatibility, and being capable of radial expansion and of being fixed in said expanded state.

2. The device of claim 1, wherein said flexible tube (30, 40, 50), prior to use as an endoprosthesis (20, 20') is radially shaped and reduced in diameter in such a manner that the individual meshes will be interlocking with clearance.

3. The device of claim 2 wherein said endoprosthesis formed as a radially expandable tube (30, 40, 50) comprises not less than two layers which may be differently designed, made by knitting, crocheting or the like, and encompassing each other.

4. The device of claim 3 wherein anchoring means (34) will, during radial expansion of the endoprosthesis, penetrate the outer layer of the latter.

5. The device of claim 4 wherein the said anchoring means (34) are connected with a inner layer of the endoprosthesis and wherein anchoring pins are radially extending from the latter.

6. The device of one of claims 1 through 5 wherein the tubularstructure (30, 40, 50) forming the endoprosthesis is made of filament material consisting of not less than two single filaments combined to form a cord and jointly consisting of single filaments processed by knitting, crocheting or the like.

7. The device of one of claims 2 through 6 wherein a plastic deformation of the filament material forming the flexible tubular structure (30, 40, 50) takes place within the area of the meshes during radial expansion of the endoprosthesis.

8. The device of one of claims 1 through 7 wherein part of the filament material of which the flexible tubular structure (30, 40, 50) is made can be of hollow material provided with holes or perforations as may be used for a continuous release of medicaments.

9. The device of one of claims 1 through 8 wherein the filament material of which the flexible tubular structure (30, 40, 50), forming the endoprosthesis is provided with a coating of a material of good biocompatibility.

10. The device of one of claims 1 through 9 wherein the flexible tubular structure (30, 40, 50) produced by knitting, crocheting or the like is provided, inside and/or outside, with a cuff-like covering of flexible material, as Latex.

11. The device of one of claims 1 through 10, wherein the endoprosthesis is formed as a vascular valve with tubular segments (43, 44) coaxially extending along both sides of and beyond the tubular structure (40) made by knitting, crocheting or the like and which are fitted, at their ends, with non-expandable retaining rings (45, 46), one of the segments (44) being provided with a lining or coating of Latex or another suitable material.

12. Device of one of claims 1 through 11, wherein the endoprosthesis is designed as a semilunar valve (50) or as an aortic valve, for example, with not less than two rhomboid valves arranged at one end of a tube section made by knitting, crocheting or the like, the valves consisting of a finemeshed web whose interwoven filaments run approximately parallel with each of two rhomboid edges, the valves being taken up in triangular segments of the tube section, with one each of the diagonals running approximately in the axial direction of the tube section and being connected alongside the two rhomboid edges with the edges of the triangular segments in the tube section, as by sewing up or crocheting with the aid of spring wire.

13. The device of one of claims 1 through 12 wherein, to prevent the formation of a step in the region between catheter tip and balloon section (15), a catheter with a cone (19) slightly rising to the latter is used and which forms an axial limitation in the direction of the catheter tip for the endoprosthesis mounted on the balloon section in the nonexpanded state.

14. The device of claim 13 wherein said cone is formed as a double cone (19) with one section tapered toward said balloon section (15).

**Revendications**

1. Dispositif pour la réalisation ou la dilatation de liaisons allant vers des cavités corporelles, ou entre des cavités corporelles, à l'aide d'un cathéter qui possède une partie ballonnet, pouvant subir une dilatation radiale dans une lumière sous l'effet de l'application d'un fluide sous pression, et avec une endoprothèse, logée sur la partie ballonnet du cathéter et pratiquement maintenue en position dans le sens axial, endoprothèse qui peut subir une dilatation radiale et qui, après une mise en place convenablement effectuée, peut être séparée du cathéter lors de l'affaissement de la partie ballonnet, en particulier pour ouvrir ou élargir des vaisseaux, caractérisé en ce que l'endoprothèse (20, 20') est un tuyau souple (30, 40, 50), réalisé par tricotage, au crochet ou par un autre type de formation de mailles, constitué d'un matériau à base de fils metalli- ques ou plastiques présentant une bonne compatibilité avec les tissus, pouvant subir une dilatation radiale et pouvant ensuite se fixer en position dilatée.

2. Dispositif selon la revendication 1, caractérisé en ce que le tuyau souple (30, 40, 50) est, avant son utilisation comme endoprothèse (20, 20'), soumis à une déformation radiale et à une réduction du diamètre telles que les différentes mailles s'interpénètrent, avec jeu.

3. Dispositif selon la revendication 2, caractérisé en ce que l'endoprothèse, constituée comme un tuyau souple radialement dilatable (30, 40, 50) est constitué d'au moins deux couches qui s'entourent l'une l'autre, réalisées par tricotage, au crochet ou analogue, et ayant éventuellement des constitutions différentes.

4. Dispositif selon la revendication 3, caractérisé par des moyens d'ancrage (34) qui, lors de la dilatation radiale de l'endoprothèse, en traversent la couche extérieure.

5. Dispositif selon la revendication 4, caractérisé en ce que les moyens d'ancrage (34) sont relies à une couche intérieure de l'endoprothèse et que sont présentes des tiges d'ancrage (36), qui s'en éloignent radialement.

6. Dispositif selon l'une des revendications 1 à 5, caractérisé en ce que le tuyau (30, 40, 50) formant l'endoprothèse est réalisé en un matériau du type fil, lequel, pour sa part, est constitué d'au moins deux fils individuels, réunis en cordon et mis en oeuvre en commun par tricotage, au crochet ou analogues.

7. Dispositif selon l'une des revendications 2 à 6, caractérisé par une déformation plastique du matériau du type fil formant le tuyau souple (30, 40, 50) dans la zone des mailles lors de la dilatation radiale de l'endoprothèse.

8. Dispositif selon l'une des revendications 1 à 7, caractérisé en ce que, pour au moins une partie du matériau du type fil dont est réalisé le tuyau souple (30, 40, 50), il s'agit d'un matériau creux, pourvu de trous ou de perforations, par exemple pour une administration continue de médicaments.

9. Dispositif selon l'une des revendications 1 à 8, caractérisé en ce que la matériau du type fil dont est réalisé le tuyau souple (30, 40, 50) formant l'endoprothèse est pourvu d'une enduction en un matériau compatible avec les tissus.

10. Dispositif selon l'une des revendications 1 à 9, caractérisé en ce que le tuyau souple (30, 40, 50) réalisé par tricotage, au tricot ou analogue, est pourvu sur sa face extérieure et/ou intérieure d'un enrobage de type manchette en un matériau dilatable, comme le latex.

11. Dispositif selon l'une des revendications 1 à 10, caractérisé par le fait que l'endoprothèse est constituée comme une valvule vasculaire, avec des parties tubulaires (43, 44), qui, en leurs deux extrémités s'étendent axialement au-delà du tuyau (40) réalisé par tricotage, au crochet ou d'une manière analogue, parties tubulaires pourvues en leurs extrémités de bagues de fixation non dilatables (45, 46), l'une des parties tubulaires (44) étant pourvue d'un revêtement ou d'une enduction étanche aux liquides, en latex ou en un autre matériau approprié.

12. Dispositif selon l'une des revendications 1 à 11, caractérisé en ce que l'endoprothèse est réalisée sous la forme d'une valvule sigmoïde (50), par exemple d'une valvule aortique, avec au moins deux valvules (51) en forme de losange, disposées en une extrémité d'une partie tubulaire (52) réalisée par tricotage, au crochet ou d'une manière analogue, les valvules étant constituées d'un tissu à fines mailles, dont les fibres entrelacées courent d'une manière approximativement parallèle à deux côtés du losange, les valvules étant logées dans des évidements triangulaires de la partie tubulaire, avec dans chaque cas une diagonale courant approximativement dans la direction axiale de la partie tubulaire, et, le long de deux côtés du losange, étant reliées aux côtés des évidements triangulaires aménagés dans la partie tubulaire, par exemple par couture ou au crochet à l'aide d'un fil élastique.

13. Dispositif selon l'une des revendications 1 à 12, caractérisé en ce que, pour éviter un étagement entre l'extrémité du cathéter et la partie ballonnet (15), il est prévu un cône (19), légèrement croissant vers cette dernière, cône qui, simultanément, forme vers l'extrémité du cathéter une limitation axiale pour l'endoprothèse logée, à l'état non dilaté, sur la partie ballonnet.

14. Dispositif selon la revendication 13, caractérisé en ce que le cône est réalisé sous la forme d'un double cône (19) avec une partie qui se rétrécie vers la partie ballonnet (15).

Fig. 1

Fig. 2

Fig. 3

Fig. 4

Fig. 5

Fig. 6

Fig. 7

Fig. 8

Fig. 9

Fig. 10

Fig. 11

EP 0 292 587 B1

Fig. 14

Fig. 12

Fig. 13

Fig. 15

Fig. 16

Fig. 17

Fig. 18

Fig. 19